# EUROPEAN PATENT SPECIFICATION

(11) **EP 0 412 604 B1**
(45) Date of publication and mention of the grant of the patent: **04.08.1993**
(21) Application number: 90202096.5
(22) Date of filing: 01.08.1990
(51) Int. Cl.: A61K 35/78, A23L 1/10, A23L 1/00, A61K 9/00

(54) **Agglomerated psyllium husk containing edible acid**
Essbare Säure enthaltende agglomerierte Psyllium-Spelze
Glume de psyllium aggloméré contenant d'acide comestible

(30) Priority: 10.08.1989 US 391915
(43) Date of publication of application: 13.02.1991
(73) Proprietor: THE PROCTER & GAMBLE COMPANY, Cincinnati, Ohio 45202 (US)
(72) Inventor: Barbera, Melvin Anthony, Cincinnati, Ohio 45231 (US)
(74) Representative: Canonici, Jean-Jacques

(56) References cited:
- EP-A- 0 101 891
- EP-A- 0 142 601
- WO-A-85/01441
- GB-A- 2 067 402

## Description

The present invention relates to agglomerated psyllium husk comprising edible acid (e.g., citric acid) uniformly dispersed throughout the agglomerating coating. This modification to agglomerated psyllium husk improves the mixability and dispersibility of the psyllium husk in a liquid. The present invention also relates to processes for manufacturing the agglomerated psyllium husk of the present invention.

Products containing psyllium seed husk are known (e.g., Metamucil®, sold by The Procter & Gamble Company). Such products are useful for the benefit of normalizing bowel function and laxation. In addition, recent research has demonstrated the effectiveness of psyllium seed husk fiber in reducing human serum cholesterol levels and in controlling blood glucose levels in diabetics.

Psyllium seed husk contains natural mucilage. It forms a gelatinous mass on contact with water, and it exhibits poor dispersibility and mixability in water. The psyllium husk particles tend to agglomerate when mixed with water. Hydration takes place over the surface of such agglomerated aggregates to form gel-coated lumps, the interiors of which are still substantially dry. These lumps are extremely difficult to disperse.

One way of reducing these problems while improving the taste of the psyllium product has been to use high percentages of sugar in the drink mix. The dispersibility and mixability are improved, but diabetics and people on reduced calorie diets may have difficulty taking such products in view of the high sugar content.

U.S. Patent No. 4,321,263, to Powell et al., issued March 23, 1982, discloses a method of improving the dispersibility of psyllium powder. It is described therein to wet the psyllium particles with an alcoholic solution of at least one of polyethylene glycol and polyvinylpyrrolidone and granulating the thus-coated particles.

U.S. Patent 4,551,331, to Rudin, issued November 5, 1985, describes a modified dry dietary fiber product which is said to be readily dispersible in liquids. The dry dietary fiber product (e.g., psyllium) comprises a coating of from 0.05 to 20% of a food grade emulsifier. The processes for making such products are said to comprise blending the dietary fiber product materials with the mixture of a non-toxic solvent in a food grade emulsifier followed by removing the solvent. Examples 5 and 6 illustrate aspartame-containing compositions to be dispersed in water which contain citric acid and coated psyllium.

U.S. Patent 4,459,280, to Colliopoulos et al., issued July 10, 1984, and U.S. Patent 4,548,806, to Colliopoulos et al., issued October 22, 1985, describe improving mixability and dispersibility of psyllium mucilloid by applying a film of hydrolyzed starch oligosaccharide, a mono- or di-saccharide, a polyglucose, or a polymaltose to the psyllium. Preferred therein is agglomerating the psyllium mucilloid. One suitable polyglucose described therein is polydextrose, said to be "a partially metabolizable, water-soluble polymer prepared by the condensation of a melt which consists of approximately about 89% D-glucose, about 10% sorbitol and about 1% citric acid on a weight basis."

Thus, while there has already been much research devoted to improving the dispersibility of psyllium fiber in liquids, there continues to be a need for improved products and processes for obtaining readily dispersible psyllium fiber. It has been surprisingly discovered that uniformly dispersing an edible acid throughout the agglomerating coating applied to psyllium husk improves mixability, dispersibility, and product aesthetics, as well as in some cases storage stability, for psyllium husk products having low (less than about 20%) sugar content.

It is therefore an object of the present invention to provide improved agglomerated psyllium husk comprising an agglomerating coating throughout which is uniformly dispersed an edible acid. It is a further object to provide agglomerated psyllium husk having improved mixability and dispersibility in a liquid, especially water. A further object is to provide such agglomerated psyllium husk having good aesthetics and easy preparation as drinks. An object is also to provide low calorie agglomerated psyllium husk, and low calorie psyllium-containing drink mixes, having low (less than about 20%) sugar content. Additionally, an object is to provide agglomerated psyllium-containing products having improved storage stability. Finally, an object is to provide processes for producing agglomerated psyllium husk and psyllium-containing drink mixes.

These and other objects of the present invention will become readily apparent from the detailed description which follows.

All percentages and ratios used herein are by weight unless otherwise specified. Screen mesh sizes used herein are based on U.S. standards.

The present invention relates to agglomerated psyllium husk. Said agglomerated psyllium husk comprises: (a) from 25% to 99% psyllium husk; (b) from 0.5% to 20% of agglomerating material coating on said psyllium husk; and (c) from 0.5% to 20% of edible acid uniformly dispersed throughout the agglomerating material coating on said psyllium husk, and wherein further said agglomerated psyllium husk comprises less than about 20% sugar.

The present invention further relates to processes for producing agglomerated psyllium husk according to the present invention. Said processes comprise the steps of: (a) coating to agglomerate a psyllium-containing blend with a solution mixture comprising one or more agglomerating materials and one or more edible acids, (b) drying the agglomerated psyllium husk; and (c) optionally, repeating steps (a) and (b), but at least as many times as necessary to have said agglomerated psyllium husk comprise at least about 0.5% of said edible acid uniformly dispersed throughout the agglomerating material coating on said psyllium husk.

It has been surprisingly discovered that the mixability and dispersibility, as well as aesthetics and/or storage stability, of agglomerated psyllium husk is improved when edible acid is dispersed uniformly throughout the agglomerating material coating on psyllium husk. Benefits of the same type and/or degree are not observed when none of the edible acid is uniformly dispersed throughout the agglomerating material coating (e.g., as occurs when the edible acid is simply dry blended with agglomerated psyllium husk).

The psyllium husk used in the present invention is from psyllium seeds, from plants of the Plantago genus. Various species such as Plantago lanceolate, P. rugelii, and P. major are known. Commercial psyllium husk include the French (black; Plantago indica), Spanish (P. psyllium) and Indian (blonde; P. ovata). Indian (blonde) psyllium husk is preferred for use herein. Also preferred is psyllium husk which is at least about 85% pure, more preferably at least about 90% pure, and most preferably at least about 95% pure. Compositions of the present invention comprise from 25% to 99% psyllium husk, preferably from 50% to 98%, and more preferably from 50% to 90%.

The psyllium husk is obtained from the seed coat of the psyllium seeds. It is typical to remove the seed coat from the rest of the seed by, for example, slight mechanical pressure, and then to use only the seed coat. The seed coat is preferably removed and sanitized by methods known in the art. Preferred is sanitized psyllium seed husk having substantially intact cell structure, the sanitization having been accomplished by methods such as ethylene oxide sanitization and superheated steam sanitization (as taught in EP-A-0 308,003, published March 22, 1989 by The Procter & Gamble Company). It is also preferred that the psyllium husk used herein have reduced particle size. Preferably the particle size of the psyllium husk is such that more than about 90% of the psyllium husk passes through about 40 mesh screen, and more preferably such that essentially all passes through about 80 mesh screen.

The agglomerating materials useful herein are known, having been described in detail in U.S. Patent 4,548,806 and U.S. Patent 4,459,280, both to Colliopoulos et al. These agglomerating materials are selected from the group consisting of water dispersible hydrolyzed starch oligosaccharide, mono-saccharide, di-saccharide, polyglucose, polymaltose, and mixtures thereof. Compositions of the present invention comprise from 0.5% to 20% of agglomerating material coating on said psyllium husk, preferably from 1% to 10%, and more preferably from 1% to 5%.

Starches consist of granules separated from edible sources such as potato, arrowroot, oats, wheat, peas, beans, rice, corn, buckwheat, tapioca, rye or barley. A preferred source of starch is corn. The granules exist as a polymeric compound consisting of about 27% linear polymer (amylose) and 73% branched polymer (amylopectin), with these two polymers so associated in the crystal lattice that they are practically insoluble in cold water or alcohol. Starch is soluble in boiling water giving a colloidal solution which may form a jelly on cooling.

Hydrolysis of starch may be accomplished by a reaction of either acid, enzymes (e.g., alpha-amylase, beta-amylase or amyloglucosidase), or a combination of the two either together or reacted in series. The hydrolysis will follow different pathway depending on whether acids or enzymes are used. The result is a mixture of oligosaccharides which may be separated for their different properties. The resulting separated water dispersible (preferably soluble) hydrolyzed starch oligosaccharides are classified by their reducing sugar content, i.e., the mono- or di-saccharides such as glucose or fructose. The percent reducing sugar content in the particular hydrolyzed starch oligosaccharide is measured on a weight/weight basis as the Dextrose Equivalent (or "D.E."). Hydrolyzed starch oligosaccharides with a D.E. of from 0 to 20 are called maltodextrins. The solid maltodextrins have low to moderate sweetness, low to moderate hygroscopicity, solubility in water and alcohol, and have reduced browning. Above a D.E. of about 20 the hydrolyzed starch oligosaccharides are called syrup solids. The syrup solids are soluble but have a more noticeable sweetness and are more hydroscopic. Above a D.E. of about 30, the syrup solids become less desirable for use herein. A preferred water dispersible hydrolyzed starch oligosaccharide therefore has a D.E. of from 0 to 30. A preferred maltodextrin has a D.E. of from 5 to 20, more preferably about 10 (i.e., a reducing sugar content ratio of 10% w/w of the oligosaccharide).

The mono-saccharides are those carbohydrates that in general are aldehyde-alcohols or ketone alcohols that are a hexose or pentose and have a sweet taste. They are readily soluble in water and form crystalline solids. Examples of the mono-saccharides are dextrose, mannose and fructose. The di-saccharides are those carbohydrates which yield two mono-saccharides on hydrolysis. Examples of di-saccharides are lactose, sucrose and maltose.

Polyglucose and polymaltose are those compounds exemplified by U.S. Patent Nos. 3,766,165 and 3,876,794. A commercially available preparation of a polyglucose is called polydextrose and has a low calorie content (1 Kcal/gram) and little or no sweetness. It is primarily used as a low calorie, bulk replacement for sugar in foodstuffs. Polydextrose is a partially metabolizable, water-soluble polymer prepared by the condensation of a melt which consists of approximately 89% D-glucose, about 10% sorbitol and about 1% citric acid on a weight basis.

The term "edible acids", as used herein, means any water soluble acid material having a pKₐ of less than about 5 and is safe for ingestion by humans. Examples of edible acids include, but are not limited to, citric acid, ascorbic acid, malic acid, succinic acid, tartaric acid, phosphoric acid, mono-potassium phosphate, and mixtures thereof. Preferred are ascorbic acid and citric acid, with citric acid being most preferred. The agglomerated psyllium husk of the present invention comprises from 0.5% to 20% edible acid, preferably from 1% to 10%, and more preferably from 1% to 5%, uniformly dispersed throughout the agglomerating material coating on the psyllium husk.

It is to be noted that for purposes of the present invention, not all of the edible acid must be uniformly dispersed throughout the agglomerating material coating on the psyllium husk. However, it is necessary that at least about 0.5% edible acid is so uniformly dispersed. The term "uniformly dispersed", as used herein, means that the edible acid is essentially dissolved in the coating, rather than being present as discrete masses of edible acid mixed with the agglomerated psyllium or stuck fast as discrete masses by the coating to the psyllium. Furthermore, while it is preferred that at least 0.5% edible acid be uniformly dispersed throughout all the coating, it is also acceptable to have this edible acid uniformly dispersed throughout only part of the coating, e.g., as might occur if several thin layers of coating were applied (e.g., by alternating spraying and drying) and the edible acid was uniformly dispersed in one or a few, but not all, of these layers.

In addition, the agglomerated psyllium husk prepared according to the present invention (and low calorie psyllium-containing drink mixes comprising this husk) comprises less than about 20% sugar. This is important not only to provide the preferred reduced calorie agglomerated psyllium husk and drink mixes, but also because drink mixes comprising psyllium husk having levels of sugar higher than about 20% are expected to be sufficiently mixable and dispersible in a liquid so that the addition of an edible acid to the coating on the psyllium husk would provide little noticeable benefit to the mixability of such compositions (although some small benefit may even be observed for products having sugar in the range of about 30 to 50%). The term "sugar", as used herein, means mono-saccharides and di-saccharides as described hereinbefore as suitable agglomerating materials, whether or not such materials are coated on the psyllium husk or are otherwise present in the compositions.

The processes of the present invention comprise the steps of (a) coating to agglomerate a psyllium-containing blend, preferably a dry blend, with a solution mixture comprising one or more agglomerating materials (as described hereinbefore) and one or more edible acids (also as described hereinbefore); (b) drying the agglomerated psyllium husk; and (c) optionally, repeating steps (a) and (b). The repeating of steps (a) and (b) indicated in step (c) is only optional, however, if one coating and drying step is sufficient to uniformly disperse at least about 0.5% of the edible acid throughout the agglomerating material coating on the psyllium husk, otherwise it is necessary to repeat steps (a) and (b) at least as many times as necessary to attain at least this level of edible acid uniformly dispersed. Agglomeration techniques are described in the hereinbefore referenced U.S. patents, but preferred is multiple layer coating of the psyllium husk using techniques which result in agglomerating the psyllium husk, e.g., as described in detail in U.S. Patents 4,459,280 and 4,548,806, to Colliopoulos et al.; and especially preferred is single layer coating of the psyllium husk in a single pass apparatus whereby an agglomerating material (especially maltodextrin) is applied as a single coating such that from 5% to 20% of water is applied to the psyllium husk during the coating process.

Multiple layer coating of the psyllium husk is accomplished, for example, by using fluid bed agglomerating equipment. An example of such fluid bed agglomerating equipment is the Fluid Air, Inc., Model 0300 Granulator-Dryer. Preferred single layer coating of the psyllium husk is achieved by utilizing equipment (referred to herein as single pass fluidizing powder wetting apparatus) which operates preferably by dropping a dry blend psyllium-containing material through a highly turbulent annular zone formed by a cylindrical wall and a rotating shaft with variously pitched attached blades. An agglomerating material-containing solution is sprayed into this zone to contact the dry psyllium-containing blend. The resulting coated and agglomerated psyllium husk is dropped to a fluid bed dryer where the added solvent is removed. An example of this equipment is the Bepex Turboflex Model No. TFX-4 (sold by Bepex Corporation; Minneapolis, Minnesota) with a six square foot bed vibrating fluid bed dryer (sold by Witte Corporation, Inc.; Washington, New Jersey).

The psyllium-containing blend preferably comprises from 25% to 100% of psyllium. Optional components for the psyllium-containing blend include, but are not limited to, flavoring agents, sweetening agents (preferably low calorie sweetening agents), coloring agents, agglomerating materials (especially maltodextrin) and/or pharmaceutical agents. As noted hereinbefore, it is preferred that the psyllium-containing blend be dry, but it is possible to utilize suitable solvents (e.g., alcohols and/or water) if one is careful, especially if water is utilized, not to cause substantial hydration and swelling of the psyllium, since this is expected to adversely affect the rate at which psyllium husk can interact with water or other fluids.

The solution mixture comprising one or more agglomerating materials and one or more edible acids will be prepared by selecting a liquid (e.g., alcohol and/or water) as appropriate for the agglomerating materials and edible acids being coated onto the psyllium husk. However, it is preferred that water be utilized. Preferred is also spraying the solution mixture onto a dry psyllium-containing blend. Preferably, when a spraying technique is used, the solution mixture is an aqueous solution comprising from 5% to 60% (preferably from 5% to 45%; more preferably from 10% to 30%) of agglomerating material and from 0.5% to 50% (preferably from 5% to 30%) of edible acid. It is further preferred that, when maltodextrin is utilized as the agglomerating material and citric acid is utilized as the edible acid, the ratio of maltodextrin to citric acid is within the range of from 1:10 to 10:1, preferably from 1:5 to 5:1. It is also optionally possible to repeat the coating and drying steps of the present process, thereby building up a coating on the psyllium husk which comprises several thin layers of the agglomerating material/edible acid. In addition, other optional materials may be present in the solution mixture, such as sweetening agents (preferably low calorie sweetening agents), flavoring agents, coloring agents, pharmaceutical agents, and mixtures thereof.

While it is desirable to prepare the agglomerated psyllium husk according to the present invention such that the product collected after the coating is completed is ready for ingestion by mixing it in a liquid, it is also possible to add additional materials (e.g., sweetening agents; flavoring agents; coloring agents; agglomerating materials; edible acids; pharmaceutical agents; mixtures thereof) to the agglomerated psyllium husk to provide the psyllium-containing drink mix product. Typically these additional materials (e.g., in amounts from 0.01% to 75%) would be added by dry blending or mixing with the agglomerated psyllium husk (e.g., from 25% to 99.99%), but any method which does not substantially adversely affect the mixability of the agglomerated psyllium husk may be used. Furthermore, optionally, it is preferred that agglomerates of psyllium husk having particle size less than about 80 mesh screen be milled such that essentially all of the agglomerates are smaller than about 40 mesh screen, and from 10% to 40% of the agglomerates are smaller than about 120 mesh screen.

As noted hereinbefore, the compositions of the present invention optionally comprise agents which may be added as a part of the coating and/or as a part of the psyllium-containing blend and/or added to the agglomerated psyllium husk. Preferred is low calorie sweetening agents including, but not limited to, aspartame, saccharine, cyclamate, acesulfame, and mixtures thereof. Another preferred optional component is flavoring agents. Especially preferred are those flavoring agents which are compatible with the edible acid chosen for the coating of the psyllium husk. Additional optional components are pharmaceutical agents such as, for example, aspirin, non-steroidal anti-inflammatory agents or sennosides.

The following examples further describe and demonstrate embodiments within the scope of the present invention. These examples are given solely for the purpose of illustration and are not to be construed as limitations of the present invention.

### EXAMPLE I

A psyllium-containing dry blend (85.42 parts by weight) comprising 62.96 parts by weight superheated steam sanitized psyllium husks (particle size of 98% minimum through 100 mesh screen) and 22.46 parts by weight Maltrin M100 (maltodextrin having a D.E. of about 10; sold by Grain Processing Corporation; Muscatine, Iowa) is agglomerated by spraying the dry blend with a maltodextrin-containing solution (13.65 parts by weight) comprising 1.82 parts by weight Maltrin M100, 3.64 parts by weight citric acid, and 8.19 parts by weight water using a Bepex Turboflex Model No. TFX-4 (sold by Bepex Corporation; Minneapolis, Minnesota) and then drying using a six square foot bed vibrating fluid bed dryer (sold by Witte Corporation, Inc.; Washington, New Jersey). By weight of the dried agglomerates thus produced, this process applies 9.0% water to the dry psyllium-containing blend, and the dried agglomerates have 2.0% of a maltodextrin single layer coating and 4.0% of citric acid uniformly dispersed in this maltodextrin coating. The agglomerated psyllium husk is then passed through a 10 mesh screen to break up any loose clumps and remove traces of coarse material that may have formed.

A flavored psyllium-containing drink mix is prepared using 90.88 parts by weight of the agglomerated psyllium husk, 0.83 parts by weight aspartame, 4.33 parts by weight citric acid, 3.86 parts by weight flavoring agent , and 0.10 parts by weight coloring agent.

The agglomerated psyllium husk, and the flavored agglomerated psyllium husk-containing drink mix, are readily mixable and dispersible in water. Ingestion of a liquid drink prepared by mixing one teaspoon of the drink mix in 200 ml (8 ounces) of water is effective for providing laxative benefits.

A readily dispersible and suspendable agglomerated psyllium husk product is also prepared by passing the agglomerated psyllium husk through a 40 mesh screen. The overs are reduced in size by passing through a Comitrol Processor Model 1700 (sold by Urschel Laboratories Incorporated; Valparaiso, Indiana), and the resulting material is fed back to the 40 mesh screening operation on a continuous basis. 100% of the agglomerated psyllium husk is thereby made to pass through 40 mesh screen, and about 30% of the agglomerated psyllium husk has particle size less than about 120 mesh screen. This agglomerated psyllium husk is then used in the drink mix as described hereinbefore.

## Claims

1. Agglomerated psyllium husk comprising :
(a) from 25% to 99% psyllium husk;
(b) from 0.5% to 20% of agglomerating material coating on said psyllium husk; and
(c) from 0.5% to 20% of edible acid uniformly dispersed throughout the agglomerating material coating on said psyllium husk,
and wherein further said agglomerated psyllium husk comprises less than 20% sugar.

2. Agglomerated psyllium husk according to Claim 1 wherein said edible acid is selected from citric acid, ascorbic acid, malic acid, succinic acid, tartaric acid, phosphoric acid, mono-potassium phosphate, and mixtures thereof.

3. Agglomerated psyllium husk according to Claims 1 or 2 wherein said agglomerating material coating on said psyllium husk is selected from water dispersible hydrolyzed starch oligosaccharide, monosaccharide, di-saccharide, polyglucose, polymaltose, and mixtures thereof.

4. Maltodextrin-coated agglomerated psyllium husk according to any of Claims 1-3 comprising :
(a) from 50% to 98% psyllium husk of particle size such that more than 90% of the psyllium husk passes through 40 mesh screen;
(b) from 1% to 10% maltodextrin coating on said psyllium husk; and
(c) from 1% to 10% citric acid uniformly dispersed throughout the maltodextrin coating on said psyllium husk;
and wherein further said agglomerated psyllium husk comprises less than 20% sugar.

5. Agglomerated psyllium husk according to any of Claims 1-4 comprising maltodextrin having a D.E. within the range of 5 to 20.

6. Agglomerated psyllium husk according to any of Claims 1-5 further comprising sennoside.

7. Processes for producing agglomerated psyllium husk comprising the steps of :
(a) coating to agglomerate a psyllium containing-blend with a solution mixture comprising one or more agglomerating materials and one or more edible acids;
(b) drying the agglomerated psyllium husk; and
(c) optionally, repeating steps (a) and (b), but at least as many times as necessary to have said agglomerated psyllium husk comprise at least 0.5% of said edible acid uniformly dispersed throughout the agglomerating material coating on said psyllium husk.

8. Processes according to Claim 7 wherein the edible acid is selected from citric acid, ascorbic acid, malic acid, succinic acid, tartaric acid, phosphoric acid, mono-potassium phosphate, and mixtures thereof.

9. Processes according to Claims 7 or 8 wherein an aqueous mixture of the edible acid and the agglomerating material is sprayed onto a dry psyllium-containing blend to form agglomerates; and wherein said agglomerating material is selected from water dispersible hydrolyzed starch oligosaccharide, mono-saccharide, di-saccharide, polyglucose, polymaltose, and mixtures thereof.

10. Processes according to any of Claims 7-9 wherein the agglomerating material comprises maltodextrin and the edible acid comprises citric acid.

## Patentansprüche

1. Agglomerierte Psylliumschalen, welche umfassen:
(a) 25 % bis 99 % Psylliumschalen;
(b) 0,5 % bis 20 % eines Überzuges aus einem agglomerierenden Material auf den Psylliumschalen; und
(c) 0,5 % bis 20 % einer eßbaren Säure, welche innerhalb des Überzuges aus dem agglomerierenden Material auf den Psylliumschalen gleichförmig dispergiert ist;
und wobei diese agglomerierten Psylliumschalen weiterhin weniger als 20 % Zucker enthalten.

2. Agglomerierte Psylliumschalen nach Anspruch 1, wobei die eßbare Säure aus Zitronen-, Ascorbin-, Apfel-, Bernsstein-, Wein-, Phosphorsäure, Monokaliumphosphat und Gemischen hievon ausgewählt ist.

3. Agglomerierte Psylliumschalen nach Anspruch 1 oder 2, wobei der Überzug aus dem agglomerierenden Material auf den Psylliumschalen aus einem in Wasser dispergierbaren, hydrolysierten Stärkeoligosaccharid, Monosaccharid, Disaccharid, Polyglukose, Polymaltose und Gemischen hievon ausgewählt ist.

4. Mit Maltodextrin überzogene, agglomerierte Psylliumschalen nach einem der Ansprüche 1 bis 3, welche umfassen:
(a) 50 % bis 98 % Psylliumschalen einer solchen Teilchengröße, daß mehr als 90 % der Psylliumschalen durch ein Sieb der US-Standardsiebgröße Nr. 40 hindurchgehen;
(b) 1 % bis 10 % eines Maltrodextrin-Überzuges auf den Psylliumschalen; und
(c) 1 % bis 10 % Zitronensäure, welche innerhalb des Maltrodextrin-Überzuges auf den Psylliumschalen gleichförmig dispergiert ist;
und wobei das agglomerierten Psylliumschalen weiterhin weniger als 20 % Zucker enthalten.

5. Agglomerierte Psylliumschalen nach einem der Ansprüche 1 bis 4, welche ein Maltrodextrin mit einem DE-Wert in einem Bereich von 5 bis 20 aufweisen.

6. Agglomerierte Psylliumschalen nach einem der Ansprüche 1 bis 5, welche weiterhin Sennoside enthalten.

7. Verfahren zur Herstellung von agglomerierten Psylliumschalen, welches die folgenden Stufen umfaßt:
(a) Überziehen eines Psyllium enthaltenden Gemisches mit einem Lösungsgemisch, welches ein oder mehrere agglomerierende Materialien und eine oder mehrere eßbare Säure enthält, um das Psyllium enthaltende Gemisch zu agglomerieren;
(b) Trocknen der agglomerierten Psylliumschalen; und
(c) gegebenenfalls Wiederholen der Stufen (a) und (b), aber wenigstens so viele Male, als notwendig sind, damit die betreffenden agglomerierten Psylliumschalen wenigstens 0,5 % der eßbaren Säure gleichförmig dispergiert innerhalb des Überzuges aus dem agglomerierenden Material auf den Psylliumschalen aufweisen.

8. Verfahren nach Anspruch 7, wobei die eßbare Säure aus Zitronen-, Ascorbin-, Apfel-, Bernstein-, Wein-, Phosphorsäure, Monokaliumphosphat und Gemischen hievon ausgewählt ist.

9. Verfahren nach Anspruch 7 oder 8, wobei ein wäßriges Gemisch aus der eßbaren Säure und dem agglomerierenden Material auf ein Psyllium enthaltendes Trockengemisch unter Bildung von Agglomeraten aufgesprüht wird; und wobei das agglomerierende Material aus einem in Wasser dispergierbaren, hydrolysierten Stärkeoligosaccharid, Monosaccharid, Disaccharid, Polyglukose, Polymaltose und Gemischen hievon ausgewählt ist.

10. Verfahren nach einem der Ansprüche 7 bis 9, wobei das agglomerierende Material Maltodextrin umfaßt, und wobei die eßbare Säure Zitronensäure umfaßt.

## Revendications

1. Gousse de psyllium agglomérée comprenant :
(a) 25 % à 99% de gousse de psyllium,
(b) 0,5 % à 20 % de revêtement de matière agglomérante sur ladite gousse de psyllium, et
(c) 0,5 % à 20 % d'acide comestible uniformément dispersés dans le revêtement de matière agglomérante sur ladite gousse de psyllium,
et dans laquelle , en outre, ladite gousse de psyllium agglomérée comprend moins de 20 % de sucre .

2. Gousse de psyllium agglomérée selon la revendication 1, dans laquelle ledit acide comestible est choisi parmi l'acide citrique, l'acide ascorbique, l'acide malique, l'acide succinique, l'acide tartrique, l'acide phosphorique, le phosphate monopotassique et leurs mélanges .

3. Gousse de psyllium agglomérée selon la revendication 1 ou 2 , dans laquelle ledit revêtement de matière agglomérante sur ladite gousse de psyllium est choisi parmi l'amidon hydrolysé, les oligosaccharides , les monosaccharides, les disaccharides , la polyglucose , le polymaltose et leurs mélanges dispersables dans l'eau.

4. Gousse de psyllium agglomérée revêtue de maltodextrine selon l'une quelconque des revendications 1 à 3, comprenant :
(a) 50 % à 98% de gousse de psyllium d'un calibre particulaire tel que plus de 90% des gousses de psyllium passent à travers un tamis de 40 mesh,
(b) 1 à 10 % d'un revêtement de maltodextrine sur ladite gousse de psyllium, et
(c) 1% à 10 % d'acide citrique uniformément dispersée dans le revêtement de maltodextrine sur ladite gousse de psyllium,
et dans laquelle , en outre, ladite gousse de psyllium agglomérée comprend moins de 20 % de sucre.

5. Gousse de psyllium agglomérée selon l'une quelconque des revendications 1 à 4 , comprenant de la maltodextrine ayant un D.E. dans la plage de 5 à 20 .

6. Gousse de psyllium agglomérée selon l'une quelconque des revendications 1 à 5 , comprenant par ailleurs un sennoside.

7. Procédés de production de gousses de psyllium agglomérées comprenant les étapes suivantes :
(a) on revêt , à des fins d'agglomération, un mélange contenant du psyllium par un mélange en solution comprenant une ou plusieurs matières d'agglomération et un ou plusieurs acides comestibles ,
(b) on sèche la gousse de psyllium agglomérée, et
(c) facultativement, on répète les stades (a) et (b), mais au moins autant de fois que nécessaire pour que ladite gousse de psyllium agglomérée comprenne au moins 0,5 % dudit acide comestible uniformément dispersé dans le revêtement de matière d'agglomération sur ladite gousse de psyllium.

8. Procédés selon la revendication 7, dans lesquels l'acide comestible est choisi parmi l'acide citrique, l'acide ascorbique, l'acide malique, l'acide succinique, l'acide tartrique, l'acide phosphorique, le phosphate monopotassique et leurs mélanges .

9. Procédés selon la revendication 7 ou 8 , dans lesquels un mélange aqueux de l'acide comestible et de la matière d'agglomération est pulvérisé sur un mélange sec contenant du psyllium pour former des agglomérés, et ladite matière d'agglomération est choisie parmi l'amidon hydrolysé, les oligosaccharides, les monosaccharides, les disaccharides, le polyglucose, le polymaltose et leurs mélanges dispersables dans l'eau .

10. Procédés selon l'une quelconque des revendications 7 à 9, dans lesquels la matière d'agglomération comprend la maltodextrine et l'acide comestible comprend l'acide citrique.
